# EUROPÄISCHE PATENTSCHRIFT

(11) **EP 1 489 908 B1**
(45) Veröffentlichungstag und Bekanntmachung des Hinweises auf die Patenterteilung: **01.07.2009**
(21) Anmeldenummer: 03722354.2
(22) Anmeldetag: 25.03.2003
(51) Int. Cl.: A01N 59/14

(54) **INSTRUMENTENDESINFEKTION**
DISINFECTION OF INSTRUMENTS
DESINFECTION D'INSTRUMENTS

(30) Priorität: 03.04.2002 DE 10214750
(43) Veröffentlichungstag der Anmeldung: 29.12.2004
(73) Patentinhaber: ECOLAB INC., St. Paul MN 55102-2233 (US)
(72) Erfinder: MEYER, Bernhard, 40822 Mettmann (DE); DECKER, Michael, 42697 Solingen (DE); BIERING, Holger, 41516 Grevenbroich (DE)
(74) Vertreter: Polypatent
(86) Internationale Anmeldenummer: PCT/EP2003/003065
(87) Internationale Veröffentlichungsnummer: WO 2003/082014

(56) Entgegenhaltungen:
- EP-A- 0 268 170
- EP-A- 1 064 845
- WO-A-87/06951
- WO-A-94/26862
- DE-A- 2 701 133
- DE-A- 3 615 787

## Beschreibung

Die vorliegende Erfindung betrifft ein pulverförmiges Desinfektionsmittel, umfassend ein Peroxid, ein Acylierungsmittel und nichtionische Tenside und die Verwendung derartiger Desinfektionsmittel zur Oberflächen- und Instrumentendesinfektion, insbesondere auf dem Gebiet der Medizin.

Zur chemischen Desinfektion von Instrumenten mit Hilfe wäßriger Zubereitungen sind im Laufe der Zeit zahlreiche Vorschläge gemacht worden, in denen die unterschiedlichsten antimikrobiellen Wirkstoffe für die Desinfektion vorgesehen werden. In der Praxis haben Zubereitungen auf Basis von Aldehyden die weiteste Verbreitung gefunden, doch sind auch Präparate mit quartären Ammoniumverbindungen, Phenolen, Alkoholen und anderen Desinfektionswirkstoffen in Gebrauch. Zubereitungen auf Basis peroxidischer Wirkstoffe, insbesondere von Peressigsäure haben dagegen für diese Anwendung nur geringe Bedeutung erlangt. Ein wesentlicher Grund liegt in der geringen Lagerstabilität derartiger wäßriger Zubereitungen. Wegen der breiten antimikrobiellen Wirksamkeit der Peroxide hat es nicht an Versuchen gefehlt, den Nachteil der geringen Lagerstabilität zu überwinden. So ist beispielsweise in den deutschen Offenlegungsschriften 26 55 599 und 28 15 400 vorgeschlagen worden, die für die Desinfektion benötigten wäßrigen Zubereitungen erst kurz vor Gebrauch aus stabileren Vorstufen, nämlich aus Natriumperborat und Säureanhydriden, herzustellen. Gemäß der deutschen Offenlegungsschrift 27 01 133 werden die wäßrigen Zubereitungen aus Wasserstoffperoxid-Abspaltern und aromatischen Acyloxycarbonsäuren erhalten. Nur wenige dieser Verbindungen liefern aber Desinfektionslösungen mit ausreichend breiter Wirksamkeit, und die Lagerung dieser Acylierungsmittel im Gemisch mit den nötigen anorganischen Peroxiden ist wegen Zersetzungsreaktionen auch nur begrenzte Zeit möglich. Unter der Bezeichnung Sekusept Pulver ist ein Produkt im Handel, bei dessen Auflösung in Wasser durch Umsetzung von Natriumperborat mit Tetraacetylethylendiamin (TAED) eine desinfektionswirksame Zubereitung entsteht. Dieses Produkt auf Basis einer N-Acylverbindung besitzt ein breites Wirkungsspektrum und ist lagerstabil. EP 1 064 845 A1 offenbart pulverförmige Desinfektionsmittel enthaltend ein anorganisches Peroxid, Tetraacetylethylendiamin sowie ein Salz und vorzugweise ein anionisches, kationisches oder nichtionisches Tensid wie Laurylsulfat oder Polyoxyethylen Laurylether. DE 36 15 787 A1 offenbart pulverförmige Desinfektionsmittel enthaltend als anorganisches Peroxid Magnesiummonoperoxyphthalat, gegebenfalls im Gemisch mit einem oder weiteren Sauerstoffdonatoren, und als Acyldonator ein Gemisch aus zwei oder drei der Acetyldonatoren Tetraacetylethylendiamin, Pentaacetylglucose und Tetraacetylglykoluril sowie gegebenfalls weitere Zusätze wie waschaktive Substanzen. Als Tensid werden bevorzugt Alkylpolyethylenglykolether eingesetzt. Obwohl auf diese Weise bereits ein hoher Standard bei der Desinfektion von medizinischen Instrumenten erreicht worden ist, wurde weiter an der Verbesserung der peroxidischen Systeme gearbeitet, um noch bestehende Wirkungslücken und Nachteile im Gebrauch zu beseitigen. Insbesondere besteht ein Nachteil derartiger pulvriger Systeme darin, daß sie sich in Wasser nur sehr langsam lösen. Dadurch ergibt sich einerseits der Nachteil, daß die gewünschte Desinfektionsmittelkonzentration erst sehr spät voll zur Verfügung steht. Andererseits besteht zusätzlich das Risiko, daß ungelöste Bestandteile im zu desinfizierenden System oder auf der zu desinfizierenden Oberfläche verbleiben und nicht ausgespüft werden.

Aufgabe der vorliegenden Erfindung war es daher, innerhalb kurzer Zeit eine ausreichende Desinfektionsmittelkonzentration zur Verfügung zu stellen und dabei das Risiko von Rückständen im System und auf den Oberflächen gering zu halten.

Es sollte auch erreicht werden, innerhalb kürzerer Zeit die Abtötung von Mikroorganismen, auch von Mykobakterien zu erreichen.

Diese Aufgabe wurde gelöst durch die erfindungsgemäßen Mittel.

Dementsprechend ist Gegenstand der vorliegenden Erfindung ein pulverförmiges Desinfektionsmittel auf Aktivsauerstoffbasis, enthaltend ein Peressigsäure generierendes System aus einem Peroxid und einem Acylierungsmittel zusammen mit nichtionischen Tensiden.

Dabei sind die genannten nichtionischen Tenside frei von alkoxylierten Alkylphenolen und umfassen geradkettige oder in 2-Stellung methylverzweigte Etheralkohole der Formel

R-O-(PO)₁₋₂-(EO)₆₋₈-H (I)

wobei die Alkyl- bzw. Alkenylreste R wie folgt zusammengesetzt sind:
C₈ = 0-5 Gew.%;
C₉₋₁₀ = 75-90 Gew.%;
C₁₁₋₁₂ = 5-15 Gew.%;
C₁₃₋₁₄ = 4-10 Gew.%;
C₁₅₋₁₆ = 0-3 Gew.%.

Das genannte Peroxid ist vorzugsweise ausgewählt aus der Gruppe Natriumperboratmonohydrat, Natriumperborattetrahydrat, Natriumpercarbonat und deren Mischungen.

Das genannte Acylierungsmittel ist vorzugsweise ausgewählt aus der Gruppe Tetraacetylglykoluril, Tetraacetylethylendiamin, Diacetylhexahydrotriazindion und deren Mischungen.

Selbstverständlich können auch andere N-Acylverbindungen in Frage kommen, die auch im Bereich der Waschmittelchemie als sogenannte Bleichaktivatoren zur Umsetzung mit Wasserstoffperoxid in alkalischen Waschflotten beschrieben worden sind. Geeignete N-Acylverbindungen sind insbesondere diejenigen, die an dem Stickstoff, der die Acylgruppe trägt, eine weitere Ketogruppe aufweisen, und/oder in denen der Stickstoff Teil eines heterocyclischen Ringsystems ist. Beispiele geeigneter N-Acylverbindungen sind die mehrfach acylierten Alkylendiamine, wie etwa Tetraacetylethylendiamin, acylierte Glykolurile, in erster Linie Tetraacetylglykoluril, N-acylierte Hydantoine, Hydrazide, Triazole, Triazine, Urazole, Diketopiperazine, Sulfurylamide, Lactame und Cyanurate.

Vorzugsweise enthält das erfindungsgemäße Desinfektionsmittel
10 bis 70 Gew.-%, vorzugsweise 15 bis 60 Gew.-% des genannten Peroxids,
10 bis 40 Gew.-%, vorzugsweise 15 bis 30 Gew.-% des genannten Acylierungsmittels,
0,1 bis 10 Gew.-%, vorzugsweise 0,5 bis 5 Gew.-% des genannten nichtionischen Tensids und
als Rest auf 100 Gew.-% lösliches anorganisches Salz und ggf. weitere Hilfsstoffe.

Als weitere Hilfsstoffe kommen Alkalisierungsmittel, Komplexbildner für Wasserhärte, Komplexbildner für Schwermetallionen und wasserlösliche anorganische Salze, Korrosionsinhibitoren und andere Tenside in Betracht. Die Menge an derartigen Hilfsstoffen kann in den Zubereitungen in sehr weiten Grenzen, abhängig von der beabsichtigten Wirkung, schwanken. Sie liegt üblicherweise nicht über etwa 3 Gew.-%, vorzugsweise zwischen etwa 0,001 und etwa 1 Gew.-%, bezogen auf die gesamte Zubereitung

Als Komplexbildner für die Wasserhärte ist in erster Linie Natriumtriphosphat zu erwähnen, doch kommen hierfür auch andere Polyphosphate, Salze der Nitrilotriessigsäure und Salze von organischen Polycarbonsäuren, beispielsweise Citronensäure, oder von polymeren Polycarbonsäuren, beispielsweise Acrylsäure-Maleinsäure-Copolymerisaten, in Betracht. Besonders bevorzugt wird Natriumtriphosphat, das zugleich als Alkalisierungsmittel wirkt.

Als Komplexbildner für Schwermetallionen, die zersetzend auf peroxidische Verbindungen wirken, kommen in erster Linie Aminopolycarbonsäuren bzw. deren Salze, beispielsweise Ethylendiamintetraessigsäure, insbesondere aber Aminopolyphosphonsäuren, wie Ethylendiamintetramethylenphosphonsäure, oder auch Hydroxyethandiphosphonsäure bzw. deren Salze in Betracht.

Wasserlösliche Salze können die Funktion von Füllstoffen oder Gerüststoffen übernehmen, wie beispielsweise Natriumsulfat, sofern sie nicht gleichzeitig alkalisierende Wirkung haben, wie beispielsweise Natriumcarbonat und Natriumsilikat. Als Korrosionsinhibitoren sind insbesondere Alkylphosphonsäuren zu erwähnen, von denen Octanphosphonsäure besonders bevorzugt wird. Als weitere mögliche Hilfsstoffe sind Farbstoffe, Parfüm und lösungsvermittelnde Zusätze zu erwähnen.

Bei der Anwendung wird das Desinfektionsmittel in üblicher Weise mit Wasser verdünnt.

Es wird vorzugsweise in einer Menge von 1 bis 10 Gew.-% in Wasser gelöst.

In einer bevorzugten Ausführungsform wird das erfindungsgemäße Desinfektionsmitteln für die Oberflächendesinfektion und/oder Instrumentendesinfektion verwendet.

Außerdem ist es bevorzugt, das erfindungsgemäße Desinfektionsmitteln zur Abtötung von grampositiven Bakterien, und/oder zur Abtötung von Mykobakterien und/oder zur Abtötung von Viren zu verwenden.

### Beispiele

### 1. Herstellung der Wirkstofflösung

Es wurde ausgegangen von drei unterschiedlichen pulverförmigen Gemischen, jeweils bestehend aus
50 Gew.-% Natriumperboratmonohydrat und 25 Gew.-% TAED-Pulver sowie
   a) kein Tensid b) 2 Gew.% ABS (Alkylbenzolsulfonat) und
   c) 2 Gew.% Dehydol 980

Dabei wurden als Rest auf 100 Gew.% Korrosionsinhibitoren, Komplexbildner und weitere anorganische Salze eingesetzt.

In einem ersten Versuch wurde das Auflöseverhalten dieser unterschiedlichen Pulver-Formulierungen 1a) bis 1c) in Wasser ohne Rühren oder sonstige Bewegung untersucht.

Hierfür wurden je 8 g der Pulver-Formulierungen 1a) bis 1c) in je 100 ml Leitungswasser bei Raumtemperatur gegeben.

Dabei zeigte sich, daß der Zeitaufwand, der zur Auflösung der Formulierungen 1a) und 1b) erforderlich war, über eine Stunde betrug. Hinzu kam, daß sich bei der Pulver-Formulierung 1b) außerdem ein Bodensatz bildete.

Bei der Pulver-Formulierung 1c) hingegen war das Pulver innerhalb einer Stunde gelöst. Es bildete sich außerdem kein Bodensatz.

Zur Erklärung:
Dehydol 980 ist ein in 2-Stellung methylverzweigter Etheralkohol der Formel

   R-O-(PO)₁₋₂-(EO)₆₋₈-H (I)

   wobei die Alkyl- bzw. Alkenylreste R wie folgt zusammengesetzt sind:
   C₈ = 0-5 Gew.%;
   C₉₋₁₀ = 75-90 Gew.%;
   C₁₁₋₁₂ = 5-15 Gew.%;
   C₁₃₋₁₄ = 4-10 Gew. %;
   C₁₅₋₁₆ = 0-3 Gew.%.

### 2. Prüfung der Wirksamkeit gegen das grampositive Bakterium Enterococcus hirae

Mit den Pulver-Formulierungen 1a) und 1c) wurden durch Lösen von je 12,5 g in je 100 ml Leitungswasser Anwendurigslösungen für mikrobiologischen. Untersuchungen angesetzt.

Diese Lösungen wurden im quantitativen Keimträgertest nach neuer DGHM Richtlinie unter organischer Belastung ("dirty conditions") (Stand 1.3.2001) gegen das grampositive Bakterium Enterococcus hirae geprüft. Dabei wurden folgende log-Reduktionsfaktoren (jeweils Dreifachbestimmung) ermittelt:

| Einwirkzeit | 1c) | 1a) |
|---|---|---|
| 1 min | 3,06/2,23/3,1 | 0,53/0,61/0,48 |
| 5 min | 3,22/3,81/2,98 | 1,74/1,41/1,33 |
| 10 min | 6,8/6,8/6,8 | 3,72/3,46/3,8 |

Auch bezüglich der antimikrobiellen Wirksamkeit ist somit ein deutlicher Vorteil der erfindungsgemäßen Formulierungen erkennbar.

### 3. Prüfung der Wirksamkeit gegen Poliovirus

Mit den Pulver-Formulierungen 1a) und 1c) wurden durch Lösen von je 12,5 g in je 100 ml Leitungswasser Anwendungslösungen für mikrobiologischen Untersuchungen angesetzt.

Diese Lösungen wurden im quantitativen Suspensionstes nach DVV Richtlinie gegen Poliovirus geprüft. Dabei wurden folgende durchschnittliche log-Reduktionsfaktoren (jeweils Dreifachbestimmung) ermittelt:

| Einwirkzeit | 1c) | 1a) |
|---|---|---|
| 5 min | 1,5 | 1,5 |
| 10 min | 3,3 | 2,6 |

Auch die virologischen Prüfergebnisse lassen somit einen Vorteil erkennen.

## Patentansprüche

1. Pulverförmiges Desinfektionsmittel auf Aktivsauerstoffbasis, enthaltend ein Peressigsäure generierendes System aus einem Peroxid und einem Acylierungsmittel zusammen mit nichtionischen Tensiden wobei die genannten nichtionischen Tenside frei von alkoxylierten Alkylphenolen sind und geradkettige oder in 2-Stellung methylverzweigte Etheralkohole der Formel R-O-(PO)₁₋₂-(EO)₆₋₈-H (I)
umfassen, wobei die Alkyl- bzw. Alkenylreste R wie folgt zusammengesetzt sind:
C₈ = 0-5 Gew.%;
C₉₋₁₀ = 75-90 Gew.%;
C₁₁₋₁₂ =5-15 Gew.%;
C₁₃₋₁₄ = 4-10 Gew.%_{:}
C₁₅₋₁₆ = 0-3 Gew.%.

2. Desinfektionsmittel nach einem der Anspruch 1, **dadurch gekennzeichnet, daß** das genannte Peroxid ausgewählt ist aus der Gruppe Natriumperboratmonohydrat, Natriumperborattetrahydraf, Natriumpercarbonat und deren Mischungen.

3. Desinfektionsmittel nach einem oder mehreren der Ansprüche 1 oder 2, **dadurch gekennzeichnet, daß** das genannte Acylierungsmittel ausgewählt ist aus der Gruppe Tetraacetylglykoluril, Tetraacetylethylendiamin, Diacetylhexahydrotriazindion und deren Mischungen.

4. Desinfektionsmittel nach einem oder mehreren der Ansprüche 1 bis 3, **dadurch gekennzeichnet, daß** es
10 bis 70 Gew.-%, vorzugsweise 15 bis 60 Gew.-% des genannten Peroxids,
10 bis 40 Gew.%, vorzugsweise 15 bis 30 Gew.-% des genannten Acylierungsmittels,
0,1 bis 10 Gew.-%, vorzugsweise 0,5 bis 5 Gew.-% des genannten nichtionischen Tensids und
zu 100 Gew.-% lösliches anorganisches Salz und ggf. weitere Hilfsstoffe
enthält.

5. Verwendung von Desinfektionsmitteln gemäß einem der Ansprüche 1 bis 4 zur Oberflächendesinfektion.

6. Verwendung von Desinfektionsmitteln gemäß einem der Ansprüche 1 bis 4 zur Instrumentendesinfektion.

7. Verwendung von Desinfektionsmitteln gemäß einem der Ansprüche 1 bis 4 zur Abtötung von grampositiven Bakterien.

8. Verwendung von Desinfektionsmitteln gemäß einem der Ansprüche 1 bis 4 zur Abtötung von Mykobakterien.

9. Verwendung von Desinfektionsmitteln gemäß einem der Ansprüche 1 bis 4 zur Abtötung von Viren.

## Claims

1. A powdered disinfecting composition based on active oxygen, containing a peracetic acid-generating system comprising a peroxide and an acylating agent together with non-ionic surfactants, wherein said non-ionic surfactants are free from alkoxylated alkyl phenols and comprise alcohols which are linear or methyl-branched in the 2-position according to the formula
R-O-(PO)₁₋₂-(EO)₆₋₈-H (I)
wherein the alkyl and alkenyl residues R, respectively, are composed as follows:
| | |
|---|---|
| C₈ = | 0 - 5 wt.-% |
| C₉₋₁₀ = | 75-90 wt.-% |
| C₁₁₋₁₂ = | 5-15wt.-% |
| C₁₃₋₁₄ = | 4 - 10 wt.-% |
| C₁₅₋₁₆ = | 0 - 3 wt.-% |

2. Disinfecting composition according to claim 1, **characterized in that** said peroxide is selected from the group consisting of sodium perborate monohydrate, sodium perborate tetrahydrate, sodium carbonate, and mixtures thereof.

3. Disinfecting composition according to claim 1 or 2, **characterized in that** said acylating agent is selected from the group consisting of tetraacetyl glycoluril, tetraacetyl ethylenediamine, diacetyl hexahydrotriazinedione, and mixtures thereof.

4. Disinfecting composition according to any of claim 1 to 3, **characterized in that** it contains
10 to 70 wt.-%, preferably 15 to 60 wt.-% of the above-mentioned peroxide,
10 to 40 wt.-%, preferably 15 to 30 wt.-% of the above-mentioned acylating agent,
0.1 to 10 wt.-%, preferably 0.5 to 5 wt.-% of the above-mentioned non-ionic surfactant, and
to 100 wt.-% of soluble inorganic salt and optionally further auxiliaries as remainder.

5. The use of disinfecting compositions according to any of claims 1 to 4 for disinfecting surfaces.

6. The use of disinfecting compositions according to any of claims 1 to 4 for disinfecting instruments.

7. The use of disinfecting compositions according to any of claims 1 to 4 for killing gram-positive bacteria.

8. The use of disinfecting compositions according to any of claims 1 to 4 for killing mycobacteria.

9. The use of disinfecting compositions according to any of claims 1 to 4 for killing viruses.

## Revendications

1. Désinfectant pulvérulent à base d'oxygène actif, contenant un système engendrant de l'acide peracétique à partir d'un peroxyde et d'un agent d'acylation, conjointement à des agents tensioactifs non ioniques, les agents tensioactifs non ioniques cités étant exempts d'alkylphénols alkoxylés et comportant des étheralcools, linéaires ou ramifiés avec un méthyle en 2^{ème} position, de la formule
R-O-(PO)₁₋₂-(EO)₈₋₈-H (1)
les radicaux alkyles et/ou alcényles R étant composés comme suit :
C₈ = 0-5 % en poids ;
C₉₋₁₀ = 75-90 % en poids ;
C₁₁₋₁₂ = 5-15 % en poids ;
C₁₃₋₁₄ = 4-10 % en poids ;
C₁₅₋₁₆ = 0-3 % en poids.

2. Désinfectant selon la revendications 1, **caractérisé en ce que** le peroxyde cité est choisi parmi le groupe du monohydrate de perborate de sodium, du tétrahydrate de perborate de sodium, du percarbonate de sodium et de leurs mélanges.

3. Désinfectant selon l'une quelconque des revendications 1 ou 2 ou selon les revendications 1 et 2, **caractérisé en ce que** l'agent d'acylation cité est choisi parmi le groupe du tétra-acétatylglycolurile, de la tétra-acétylènediamine, de la diacétylhexahydro-triazinedione et de leurs mélanges.

4. Désinfectant selon l'une quelconque des revendications 1 à 3 ou selon plusieurs des revendications 1 à 3, **caractérisé en ce qu'**il contient
- de 10 à 70 % en poids, de préférence, de 15 à 60 % en poids du peroxyde cité,
- de 10 à 40 % en poids, de préférence, de 15 à 30 % en poids de l'agent d'acylation cité,
- de 0,1 à 10 % en poids, de préférence, de 0,5 à 5 % en poids de l'agent tensioactif non ionique cité et
- de jusqu'à 100 % en poids d'un sel inorganique soluble et, le cas échéant, d'adjuvants supplémentaires.

5. Utilisation de désinfectants selon l'une quelconque des revendications 1 à 4 en vue de la désinfection de surfaces.

6. Utilisation de désinfectants selon l'une quelconque des revendications 1 à 4 en vue de la désinfection d'instruments.

7. Utilisation de désinfectants selon l'une quelconque des revendications 1 à 4, en vue de la destruction de bactéries Gram positives.

8. Utilisation de désinfectants selon l'une quelconque des revendications 1 à 4, en vue de la destruction de mycobactéries.

9. Utilisation de désinfectants selon l'une quelconque des revendications 1 à 4, en vue de la destruction de virus.
